# EUROPEAN PATENT APPLICATION

(11) **EP 4 574 963 A1**
(43) Date of publication of application: **25.06.2025**
(21) Application number: 23854293.0
(22) Date of filing: 09.08.2023
(51) Int. Cl.: C12N 1/20, A61K 35/747, A61K 31/728, A61P 31/04, A61P 1/00, A23L 33/135

(54) **LACTOBACILLUS RHAMNOSUS AND COMPOSITION FOR INHIBITING H. PYLORI**

(30) Priority: 15.08.2022 CN 202210974828
(71) Applicant: Bloomage Biotechnology Corporation Limited, Jinan, Shandong 250101 (CN)
(72) Inventor: ZHANG, Tianmeng, Jinan, Shandong 250101 (CN); WANG, Botao, Jinan, Shandong 250101 (CN); DU, Jing, Jinan, Shandong 250101 (CN); ZHANG, Huayue, Jinan, Shandong 250101 (CN); CUI, Shumao, Wuxi, Jiangsu 214122 (CN); ZHAI, Qixiao, Wuxi, Jiangsu 214122 (CN)
(74) Representative: Abel & Imray LLP
(86) International application number: PCT/CN2023/111868
(87) International publication number: WO 2024/037390

(57) **Abstract**

A strain of *Lactobacillus rhamnosus* and a composition comprising the *Lactobacillus rhamnosus* for inhibiting *H.Pylori.* The composition further comprises hyaluronic acid or a salt thereof. The composition can significantly reduce the colonization rate of *H.Pylori* in the stomach, thereby alleviating inflammation and gastrointestinal symptoms (GSRS rating) caused by *H.Pylori* infection, improving the rates of negative conversion and effectiveness among *H.Pylori* patients, improving the abundance of beneficial intestinal bacteria in patients, and improving the health of the gastrointestinal tracts of patients.

## Description

### TECHNICAL FIELD

The present application relates to the technical fields of food and health products, specifically to a *Lactobacillus rhamnosus* and a composition for inhibiting *Helicobacter pylori.*

### BACKGROUND

*Helicobacter pylori* (*H.Pylori*) is a microaerophilic, spiral-shaped Gram-negative bacillus. *H.Pylorii* can adhere and colonize on the gastric mucosa through adhesins, invade the body's defense system, and use the direct effect of its own toxins and the indirect effect of inducing inflammatory reactions to cause gastritis and other digestive system diseases, as well as non-digestive diseases such as ischemic cardiovascular and cerebrovascular diseases, and cerebral hemorrhage. More importantly, *H.Pylori* infection can trigger the Correa cascade reaction, causing intragastric lesions to develop from non-atrophic gastritis to atrophic gastritis, intestinal metaplasia, etc., and in severe cases, further develop into gastric cancer. Due to its non self-healing and high-risk characteristics, *H.Pylori* is considered as a Class I carcinogen by the International Agency for Research on Cancer. Antibiotic therapy is a possible treatment for patients with symptoms of infection. However, with the increase in global antibiotic resistance and the frequent occurrence of other adverse reactions, the eradication rate of *H.Pylori* continues to decrease.

In recent years, people have been mainly exploring new ways to fight *H. Pylori* using non-antibiotic substances, such as probiotics, prebiotics, plant extracts, bioactive proteins, polysaccharides, etc., in order to improve the original treatment schemes and provide new and effective treatment methods. Among them, probiotics have been widely studied due to their good biological safety, tolerance to harsh gastrointestinal environment, and the effect of antagonizing *H.Pylori.* Currently, the most studied probiotic strains against *H.Pylori* mainly comprise *Lactobacillus reuteri, Lactobacillus salivarius, Lactobacillus rhamnosus, Lactobacillus acidophilus*, *Lactobacillus plantarum*, *Lactobacillus bulgaricus* and *Bifidobacterium,* etc. Probiotics play an important role in the supplementary prevention of gastrointestinal diseases. They can be used as auxiliary therapy to minimize the use of antibiotics and their side effects, strengthen the barrier function of the mucosa, reduce inflammatory reactions, and improve patients' clinical symptoms and compliance during treatment, thereby directly or indirectly improving the eradication rate of *H.Pylori*, which is of great significance to the prevention and treatment of *H.Pylori* and related diseases.

Patent CN108741090A discloses a compound probiotic food containing prebiotics, *Bifidobacterium*, *Lactobacillus acidophilus*, *Lactobacillus paracasei* and *Lactobacillus rhamnosus* that inhibits *H.Pylori* and can be used to prevent *H.Pylori* infection. Patent WO2021/238890A1 discloses a strain of *Lactobacillus rhamnosus* that inhibits *H.Pylori,* and its *H.Pylori* clearance rate is 61.54%. Patent WO2004/031368 discloses a strain of *Lactobacillus reuteri* for treating or preventing inflammation associated with *H.Pylori.* Patent WO2013027087A1 discloses a strain of *Lactobacillus reuteri* that does not produce reuterin but can inhibit the growth of *H.Pylori* in vitro. Patent CN102174450A relates to a Lactobacillus plantarum that is acid-resistant, has an inhibitory effect on the growth and urease activity of *H.Pylori,* and has the effect of preventing or reducing the degree of infection in mice infected with *H.Pylori.* Patent WO2020/083983A1 discloses *Lactobacillus acidophilus* and *Bifidobacterium animalis* that can synergize with antibiotics to kill *H.Pylori,* but the clearance rate of *H.Pylori* has not been reported.

### SUMMARY

Chinese patent ZL2020113384895 is the applicant's previous research result, which discloses the activity of a composition comprising a hyaluronic acid and a salt thereof against *H.Pylori* digestive tract infection, wherein the molecular weight of the hyaluronic acid and a salt thereof is 800-2000 kDa, preferably 1000-2000 kDa. Animal experiments are mainly used for efficacy verification, including the effect of HA on the *H.Pylori* colonization amount in the stomach of mice, but not involving the clearance rate of *H.Pylori.*

In the prior arts, there is no research on the synergy of hyaluronic acid and probiotics against *H.Pylori,* and *anti-H.Pylori* probiotics and hyaluronic acid are mainly screened and verified through in vitro bacteriostatic tests, and less frequently through animal experiments or human experiments, and there is a lack of relevant *H.Pylori* colonization rate and clearance rate data. Based on this, the application proposes a combination of hyaluronic acid and probiotics, and verifies the anti- *H.Pylori* efficacy through animal and human experiments.

Specifically, the application adopts the following technical solutions:
1. A strain of *Lactobacillus rhamnosus,* wherein the *Lactobacillus rhamnosus* is deposited in the Guangdong Microbial Culture Collection Center with an accession number of GDMCC NO.62419.
2. The *Lactobacillus rhamnosus* according to item 1, wherein the 16s rRNA gene sequence of the *Lactobacillus rhamnosus* is shown as SEQ ID NO: 1.
3. The *Lactobacillus rhamnosus* according to item 1, which is used to inhibit *Helicobacter pylori.*
4. A composition for inhibiting *Helicobacter pylori,* comprising a hyaluronic acid or a salt thereof and *Lactobacillus rhamnosus.*
5. The composition according to item 4, wherein the salt of the hyaluronic acid is any one or two or more of sodium salt, potassium salt, magnesium salt, calcium salt, zinc salt and bismuth salt of the hyaluronic acid, preferably the sodium salt of the hyaluronic acid.
6. The composition according to item 4 or 5, the average molecular weight of the hyaluronic acid or a salt thereof is 1200-2000 kDa, preferably 1400-1800 kDa.
7. The composition according to any one of items 4-6, wherein the composition can provide the hyaluronic acid or a salt thereof with an amount of more than or equal to 80 mg/day/person, or
   the viable count of *Lactobacillus rhamnosus* is more than or equal to 1×10⁸ cfu/day/person;
   preferably, the composition can provide the hyaluronic acid or a salt thereof with an amount of more than or equal to 100 mg/day/person, or
   the viable count of *Lactobacillus rhamnosus* is more than or equal to 1×10⁹ cfu/day/person,
   more preferably, the dosage of the hyaluronic acid or a salt thereof is more than or equal to100 mg/day/person, or;
   the dosage of *Lactobacillus rhamnosus* is that the viable count of *Lactobacillus rhamnosus is* more than or equal to 1×10¹⁰ cfu/day/person.
8. The composition according to any one of items 4-7, wherein the composition further comprises an adjuvant, preferably, the adjuvant comprises one or two or more of inulin, galactooligosaccharides, isomaltooligosaccharides, maltodextrin, erythritol, xylose, arabinose, rhamnose, galactose, fucose, mannose, fructose, sorbose, glucose, glycerol, galactitol, sorbitol, xylitol, mannitol, lactose, maltitol, lactitol, sucrose, trehalose, raffinose, stachyose, oligofructose, oligoxylose, oligomannose, β-glucan, hydroxyethyl starch, resistant dextrin, whey protein, collagen, skimmed milk, pectin, and gelatin.
9. The composition according to any one of items 4-8, the dosage form of the composition may be pills, granules, powders, tablets, capsules or emulsions.
10. The composition according to any one of items 4-9, wherein the *Lactobacillus rhamnosus* is the *Lactobacillus rhamnosus* according to any one of claims 1-3.
11. Use of the *Lactobacillus rhamnosus* according to any one of items 1-3, or the composition according to any one of items 4-10 in the preparation of foods or health products inhibiting *Helicobacter pylori.*
12. Use of *Lactobacillus rhamnosus* and hyaluronic acid or a salt thereof in combination in the preparation of foods or health products inhibiting *Helicobacter pylori.*
13. The use according to item 12, the dosage of the hyaluronic acid or a salt thereof is more than or equal to 80 mg/day/person, or;
   the dosage of *Lactobacillus rhamnosus* is that the viable count of *Lactobacillus rhamnosus* is more than or equal to 1×10⁸ cfu/day/person,
   preferably, the dosage of hyaluronic acid or a salt thereof is more than or equal to100 mg/day/person, or;
   the dosage of *Lactobacillus rhamnosus* is that the viable count of *Lactobacillus rhamnosus* is more than or equal to 1×10⁹ cfu/day/person,
   more preferably, the dosage of hyaluronic acid or a salt thereof is more than or equal to 100 mg/day/person, or;
   the dosage of *Lactobacillus rhamnosus* is that the viable count of *Lactobacillus rhamnosus* is more than or equal to 1×10¹⁰ cfu/day/person.
14. The use according to item 12 or 13, the salt of the hyaluronic acid is any one or two or more of sodium salt, potassium salt, magnesium salt, calcium salt, zinc salt and bismuth salt of the hyaluronic acid..
15. The use according to any one of items 12-14, wherein the average molecular weight of the hyaluronic acid or a salt thereof is 1200-2000 kDa, preferably 1400-1800 kDa.
16. The use according to any one of items 11-15, wherein the *Lactobacillus rhamnosus* is the *Lactobacillus rhamnosus* according to any one of items 1-3.

### EFFECTS OF THE APPLICATION

1. After the composition of the hyaluronic acid or a salt thereof and *Lactobacillus rhamnosus* provided in the application is taken orally, the colonization rate of *Helicobacter pylori* in the stomach can be significantly reduced to the normal level, and the inflammatory response of the gastric mucosa and the gastrointestinal symptoms (GSRS score) of the subjects caused by *H.Pylori* are significantly improved. The negative conversion rate and effective rate of *H.Pylori* patients can reach 70.59% and 82.35%, respectively, and the abundance of beneficial intestinal flora increases, which is beneficial to improve the gastrointestinal health of patients.
2. The composition of the hyaluronic acid or a salt thereof and *Lactobacillus rhamnosus* provided in the application provides a new and effective treatment method for the prevention of H. pylori susceptible populations, the prevention of recurrence in H. pylori eradicated patients, and the treatment of asymptomatic H. pylori infected patients, etc.
3. The application provides a composition of the hyaluronic acid or a salt thereof and *Lactobacillus rhamnosus* that synergistically inhibits *H.Pylori,* which can significantly reduce the colonization rate of *Helicobacter pylori* in the stomach, alleviate the inflammatory response and gastrointestinal symptoms (GSRS score) of *H.Pylori* infection, improve the negative conversion rate and effective rate of *H.Pylori* patients, increase the abundance of beneficial intestinal bacteria in patients, and improve the gastrointestinal health of patients.

### BRIEF DESCRIPTION OF THE DRAWINGS

The accompanying drawings are used for a better understanding of the present application and do not constitute an undue limitation of the present application. wherein:

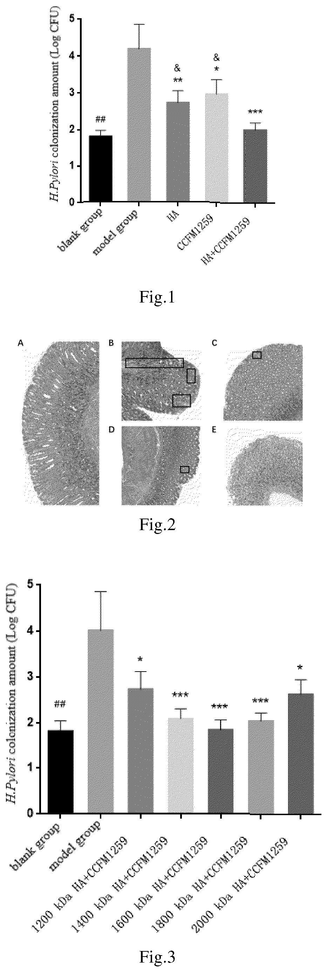

Figure 1 shows the effects of different treatments on the *H.Pylori* colonization amount in the stomach of mice; among them, * in the figure indicates there is a significant difference between the intervention group and the model group (p<0.05); ** indicates there is a significant difference between the intervention group and the model group. (p<0.01); *** indicates there is a very significant difference between the intervention group and the model group (p<0.001); & indicates there is a significant difference between the HA group, the CCFM1259 group and the compounding group (p<0.05); ## indicates there is a significant difference between the blank group and the model group (p<0.01);
Figure 2 shows the results of HE staining of the gastric mucosa of mice (200×) (A: blank group; B: model group; C: HA group; D: CCFM1259 group; E: HA+CCFM1259 compounding group);
Figure 3 shows the effect of compounding HA with different molecular weights and *Lactobacillus rhamnosus* on *H.Pylori* colonization amount in the stomach of mice; among them, * in the figure indicates there is a significant difference between the intervention group and the model group (p<0.05); ** indicates there is a significant difference between the intervention group and the model group (p<0.01); *** indicates there is a very significant difference between the intervention group and the model group (p<0.001); ## indicates there is a significant difference between the blank group and the model group (p<0.01);
Figure 4 shows the changes in GSRS in each group after the intervention (* indicates there is a significant difference compared with that before the intervention; p<0.05);
Figure 5 shows the results of subjects' intestinal flora at phylum level (A) and genus level (B).

### DETAILED DESCRIPTION

Exemplary embodiments of the present application are described below, including various details of the embodiments of the present application to facilitate understanding, and they should be considered to be exemplary only. Accordingly, those of ordinary skill in the art will recognize that various changes and modifications can be made to the embodiments described herein without departing from the scope and spirit of the application. Similarly, for clarity and conciseness, descriptions of well-known functions and structures have been omitted in the following description.

The application provides a strain of *Lactobacillus rhamnosus,* which is deposited in Guangdong Microbial Culture Collection Center (GDMCC) with an accession number of GDMCC NO.62419, and the deposit address is institute of Microbiology, Guangdong Academy of Sciences, 5th Floor, Building 59, No. 100 Xianlie Middle Road, Guangzhou, Guangdong Province, China, postal code: 510070, deposition date: May 17, 2022. Biological material name and indicated identifying characteristics: *Lactobacillus rhamnosus* CCFM1259.

The term "*Lactobacillus rhamnosus*" conforms to the general definition in this field. It belongs to the genus Lactobacillus and is one of the normal flora of the human body. It exists in the oral cavity and intestinal tract of the human body, mainly in the intestinal tract. *Lactobacillus rhamnosus* is an anaerobic, acid-resistant, and asporogenous probiotic. Its Gram stain is purple, so it is also a Gram-positive bacterium. The main functions of *Lactobacillus rhamnosus* comprise strengthening the gastrointestinal mucosal barrier, regulating the body's immunity, antagonizing pathogenic bacteria, aiding digestion, lowering blood lipids, and protecting the liver. It is a nontoxic, and has no side effects, and its functional characteristics mainly include regulating intestinal flora, preventing and treating diarrhea, expelling toxins, and enhancing the body's immunity.

The *Lactobacillus rhamnosus* provided in the application is inoculated on MRS solid plates and cultured at a constant temperature of 37°C for 48 hours. The colonies were round in shape, with neat edges, smooth and convex surfaces, uniform texture, and milky white color.

The 16s rRNA gene sequence of *Lactobacillus rhamnosus* is shown by SEQ ID NO: 1, SEQ ID NO: 1:

CTTAGACGGCTCGCTCCCTAAAGGGTTACGCCACCGGCTTCGGGTGTTACAAAC
TCTCATGGTGTGACGGGCGGTGTGTACAAGGCCCGGGAACGTATTCACCGCGGCGT
GCTGATCCGCGATTACTAGCGATTCCGACTTCGTGTAGGCGAGTTGCAGCCTACAGT
CCGAACTGAGAATGGCTTTAAGAGATTAGCTTGACCTCGCGGTCTCGCAACTCGTTG
TACCATCCATTGTAGCACGTGTGTAGCCCAGGTCATAAGGGGCATGATGATTTGACG
TCATCCCCACCTTCCTCCGGTTTGTCACCGGCAGTCTTACTAGAGTGCCCAACTAAAT
GCTGGCAACTAGTCATAAGGGTTGCGCTCGTTGCGGGACTTAACCCAACATCTCACG
ACACGAGCTGACGACAACCATGCACCACCTGTCATTTTGCCCCCGAAGGGGAAACC
TGATCTCTCAGGTGATCAAAAGATGTCAAGACCTGGTAAGGTTCTTCGCGTTGCTTC
GAATTAAACCACATGCTCCACCGCTTGTGCGGGCCCCCGTCAATTCCTTTGAGTTTC
AACCTTGCGGTCGTACTCCCCAGGCGGAATGCTTAATGCGTTAGCTGCGGCACTGAA
GGGCGGAAACCCTCCAACACCTAGCATTCATCGTTTACGGCATGGACTACCAGGGT
ATCTAATCCTGTTCGCTACCCATGCTTTCGAGCCTCAGCGTCAGTTACAGACCAGAC
AGCCGCCTTCGCCACTGGTGTTCTTCCATATATCTACGCATTTCACCGCTACACATGG
AGTTCCACTGTCCTCTTCTGCACTCAAGTTTCCCAGTTTCCGATGCACTTCCTCGGTT

AAGCCGAGGGCTTTCACATCAGACTTAAAAAACCGCCTGCGCTCGCTTTACGCCCAA
TAAATCCGGATAACGCTTGCCACCTACGTATTACCGCGGCTGCTGGCACGTAGTTAG
CCGTGGCTTTCTGGTTGGATACCGTCACGCCGACAACAGTTACTCTGCCGACCATTC
TTCTCCAACAACAGAGTTTTACGACCCGAAAGCCTTCTTCACTCACGCGGCGTTGCT
CCATCAGACTTGCGTCCATTGTGGAAGATTCCCTACTGCTGCCTCCCGTAGGAGTTT
GGGCCGTGTCTCAGTCCCAATGTGGCCGATCAACCTCTCAGTTCGGCTACGTATCAT
TGCCTTGGTGAGCCGTTACCTCACCAACTAGCTAATACGCCGCGGGTCCATCCAAAA
GCGATAGCTTACGCCATCTTTCAGCCAAGAACCATGCGGTTCTTGGATTTATGCGGT
ATTAGCATCTGTTTCCAAATGTTATCCCCCACTTAAGGGCAGGTTACCCACGTGTTA
CTCACCCGTCCGCCACTCGTTCAAAATTAAATCAAGATGCAAGCACCTTTCAATAAT
CAGAACTCGTTCGACTGCATGTATAG

The *Lactobacillus rhamnosus* provided in the application can be used to inhibit *Helicobacter pylori.*

The present application further provides a composition for inhibiting *Helicobacter pylori,* comprising a hyaluronic acid or a salt thereof and *Lactobacillus rhamnosus.*

The viable count of *Lactobacillus rhamnosus* is not less than 1×10⁸ CFU/g or 1×10⁸ CFU/ml, and preferably the viable count of *Lactobacillus rhamnosus* is not less than 1×10⁹ CFU/g or 1× 10⁹ CFU/ml, further preferably, the viable count of *Lactobacillus rhamnosus* is not less than 1×10¹⁰ CFU/g or 1×10¹⁰ CFU/ml, more preferably, the viable count of *Lactobacillus rhamnosus* is not less than 1×10¹¹ CFU/ml or 1×10¹¹ CFU/ml.

The term "hyaluronic acid" conforms to the general definition in the art and refers to a biopolymer material composed of linearly linked repeating units N-acetyl-D-glucosamine and D-glucuronic acid, also known as hyaluronic acid. It is an acidic mucopolysaccharide. Its unique molecular structure, physical and chemical properties display a variety of important functions in the body. The hyaluronic acid or a salt thereof is intended to comprise hyaluronic acid itself, a salt thereof, or a combination thereof. Examples of hyaluronates include, but are not limited to: inorganic salts such as sodium hyaluronate, potassium hyaluronate, calcium hyaluronate, magnesium hyaluronate, zinc hyaluronate, bismuth hyaluronate, and cobalt hyaluronate; and organic salts such as tetrabutylammonium hyaluronate. In the present application, hyaluronic acid itself or a salt thereof may be used alone, or a combination of two or more hyaluronic acids or a salt thereof may be used. For example, it may comprise one hyaluronate, two hyaluronates, three hyaluronates, four hyaluronates, five hyaluronates, or six hyaluronates; the types of the salts are selected from sodium salt, potassium salt, magnesium salt, calcium salt, zinc salt and bismuth salt.

The hyaluronic acid or a salt thereof in the application is not limited. In a preferred embodiment, the hyaluronate is a water-soluble salt of hyaluronic acid, further preferably any one of sodium hyaluronate, zinc hyaluronate, magnesium hyaluronate or potassium hyaluronate. In a specific embodiment, the hyaluronate is sodium hyaluronate.

In a preferred embodiment, the average molecular weight of the sodium hyaluronate is 1200-2000 kDa, for example, it may be 1200 kDa, 1300 kDa, 1400 kDa, 1500 kDa, 1600 kDa, 1700 kDa, 1800 kDa, 1900 kDa, 2000 kDa and any molecular weight there between, preferably 1400-1800 kDa.

In a preferred embodiment, in the *anti-Helicobacter pylori* composition provided by the present application, the viable count of *Lactobacillus rhamnosus* is more than or equal to 1×10⁸ cfu/day/person (a person is calculated as 60 kg). Those in the art should understand that, under normal circumstances, a person's weight is calculated as 60 kg. If the person's weight is not 60 kg, conversion can be made according to this standard. In a preferred embodiment, in the anti-*Helicobacter pylori* composition provided by the present application, the viable count of *Lactobacillus rhamnosus* is more than or equal to 1×10⁹ cfu/day/person. Those in the art should understand that, under normal circumstances, a person's weight is calculated as 60 kg. If the person's weight is not 60 kg, conversion can be made according to this standard.

In a preferred embodiment, in the *anti-Helicobacter pylori* composition provided by the present application, the dosage of the hyaluronic acid or a salt thereof is more than or equal to 80 mg/day/person. Those in the art should understand that, under normal circumstances, a person's weight is calculated as 60 kg.

In a preferred embodiment, in the *anti-Helicobacter pylori* composition provided by the present application, the viable count of *Lactobacillus rhamnosus* is more than or equal to 1×10¹⁰ cfu/day/person. Those in the art should understand that, under normal circumstances, a person's weight is calculated as 60 kg.

In a preferred embodiment, in the *anti-Helicobacter pylori* composition provided by the present application, the dosage of the hyaluronic acid or a salt thereof is more than or equal to 100 mg/day/person. Those in the art should understand that, under normal circumstances, a person's weight is calculated as 60 kg.

In a preferred embodiment, the composition further comprises an adjuvant. The adjuvant described in the application can be appropriate solvents, propellants, solubilizers, co-solvents, emulsifiers, colorants, adhesive agents, disintegrants, fillers, lubricants, wetting agents, osmotic pressure regulators, stabilizers, glidants, flavoring agents, preservatives, suspending agents, coating materials, aromatic agents, anti-adhesive agents, integrative agents, penetration enhancers, pH value regulators, buffering agents, plasticizers, surfactants, foaming agents, defoaming agents, thickeners, encapsulation agents, humectants, absorbents, diluents, flocculants and deflocculants, filter aids, release blockers, etc.

In a further preferred embodiment, the adjuvant is one or two or more of inulin, galactooligosaccharide, isomaltooligosaccharide and maltodextrin.

The compositions of the present application can be prepared by general methods, to which one or more diluents or carriers can be added, for example, in oral forms such as pills, tablets, capsules, granules, powders, lozenges, syrups, emulsions, suspensions, etc.

The application further provides use of *Lactobacillus rhamnosus* and hyaluronic acid or a salt thereof in combination for preparing foods or health products in inhibiting *Helicobacter pylori.*

The combined use may be that *Lactobacillus rhamnosus* and a hyaluronic acid or a salt thereof are mixed and used together, or it may also be that one of *Lactobacillus rhamnosus* and a hyaluronic acid or a salt thereof is agent A and the other is agent B, A is used first and then B, or B is used first and then A, or A and B can be used at the same time.

In a preferred embodiment, in the use, the dosage of the hyaluronic acid or a salt thereof is more than or equal to 80 mg/day/person. Those in the art should understand that, under normal circumstances, a person's weight is calculated as 60 kg.

In a preferred embodiment, in the use, the dosage of *Lactobacillus rhamnosus* is that the viable count of *Lactobacillus rhamnosus* is more than or equal to 1×10⁸ cfu/day/person. Those in the art should understand that, under normal circumstances, a person's weight is calculated as 60 kg.

In a preferred embodiment, in the use, the dosage of *Lactobacillus rhamnosus* is that the viable count of *Lactobacillus rhamnosus* is more than or equal to 1×10⁹ cfu/day/person. Those in the art should understand that, under normal circumstances, a person's weight is calculated as 60 kg.

In a preferred embodiment, in the use, the dosage of the hyaluronic acid or a salt thereof is more than or equal to 100 mg/day/person. Those in the art should understand that, under normal circumstances, a person's weight is calculated as 60 kg.

In a preferred embodiment, in the described use, the dosage of *Lactobacillus rhamnosus* is that the viable count of *Lactobacillus rhamnosus* is more than or equal to 1×10¹⁰ cfu/day/person. Those in the art should understand that, under normal circumstances, a person's weight is calculated as 60 kg.

In a preferred embodiment, the salt of hyaluronic acid is any one or two or more of sodium salt, potassium salt, magnesium salt, calcium salt, zinc salt and bismuth salt of the hyaluronic acid.

In a preferred embodiment, the average molecular weight of the hyaluronic acid or a salt thereof is 1200-2000 kDa, for example, it may be 1200 kDa, 1300 kDa, 1400 kDa, 1500 kDa, 1600 kDa, 1700 kDa, 1800 kDa, 1900 kDa, 2000 kDa and any molecular weight there between, preferably 1400-1800 kDa.

*Lactobacillus rhamnosus* CCFM1259 provided in the application has a good inhibitory effect on *Helicobacter pylori,* and its antibacterial effect is stronger than that of other strains, such as *Lactobacillus paracasei* TY-O1 and *Lactobacillus plantarum* SN-L3. Among them, the inhibition zone of CCFM1259 is 0.76mm and 1.67mm larger than that of the other two strains respectively, and is also better than other *Lactobacillus rhamnosus,* such as *Lactobacillus rhamnosus* TY-O4, *Lactobacillus rhamnosus* FB-O1, etc. At the same time, *Lactobacillus rhamnosus* CCFM1259 provided by the application has a better effect of reducing the adhesion rate of *H.Pylori,* which is better than *Lactobacillus paracasei* TY-O1 and *Lactobacillus plantarum* SN-L3. CCFM1259 has adhesion rates 4.86% and 8.08% lower than that of the other two strains, respectively, and its adhesion rate is also lower than that of other *Lactobacillus rhamnosus* strains such as *Lactobacillus rhamnosus* TY-O4 and *Lactobacillus rhamnosus* FB-O1, etc.

The composition for inhibiting *Helicobacter pylori,* comprising hyaluronic acid or a salt thereof and *Lactobacillus rhamnosus* provided in the application, is compounded with a hyaluronic acid or a salt thereof and *Lactobacillus rhamnosus* powder, which has a better effect on reducing the *H. Pylori* colonization amount, which is better than that of the hyaluronic acid or a salt thereof alone and *Lactobacillus rhamnosus* alone. From the experimental data of the application, it can be seen that there is a significant difference in the effect of the composition of the application compared with the hyaluronic acid or a salt thereof alone and *Lactobacillus rhamnosus* alone, (p<0.05), indicating that the composition of the application has a synergistic effect on reducing the *H.Pylori* colonization amount.

The composition for inhibiting *Helicobacter pylori,* comprising hyaluronic acid or a salt thereof and *Lactobacillus rhamnosus* provided in the application, has a better improvement in gastric pathology. Compared with the hyaluronic acid or a salt thereof alone and *Lactobacillus rhamnosus* alone, the composition of the present application has a more significant improvement effect on the gastric mucosa of mice after treatment, and there is almost no inflammatory cell infiltration. It is further proved that the composition of the present application has a synergistic effect in antagonizing the inflammatory response caused by *H.Pylori* infection.

Further, a clinical trial was conducted on the *anti-Helicobacter pylori* effect of the composition of the present application. A placebo and a composition of a hyaluronic acid or a salt thereof and *Lactobacillus rhamnosus* bacteria powder provided in the present application were administered to the subjects. Compared with the placebo group, the negative conversion rate and effective rate of the compounding group increase by 125.88% and 119.60% respectively. In the placebo group, there is no significant change in adverse gastrointestinal symptoms before and after the intervention (p>0.05); however, after the intervention of the composition, the gastrointestinal symptoms of the subjects are significantly improved (GSRS score, p<0.05), and the negative conversion rate and effective rate of the subjects are also multiplied, indicating that the composition has a significant effect in antagonizing *H.Pylori* infection in the stomach. In addition, the composition of the present application also has the function of improving the composition and diversity of intestinal flora, reducing the proportion of intestinal pathogenic bacteria, increasing the abundance of beneficial bacteria Lactobacillus and Bifidobacterium, which is beneficial for maintaining patients' gastrointestinal health.

### EXAMPLES

Animal experiment arrangements:
1) Source of the used animals
   4-week-old, male, C57BL/6 mice.
2) Information on proposed breeding facilities
   Jiangsu Institute of Parasitic Diseases.
3) Source of sodium hyaluronate (HA)
   Bloomage Biotechnology Co., Ltd., food-grade sodium hyaluronate and related products.
4) Source of probiotics
   *Lactobacillus rhamnosus* CCFM1259, deposited in the Guangdong Microbial Culture Collection Center with the accession number of GDMCC NO.62419.
5) Methods of anesthesia and execution of experimental animals

After the last intragastric administration, the mice were fasted for 24 hours, and the mice were intraperitoneally injected with a 1% sodium pentobarbital solution. After anesthesia, blood was collected from the mouse's eyeballs, and finally the mice were killed by cervical dislocation. Immediately the mice were dissected to take the stomach, cutting along the greater curvature of stomach, and taking complete gastric tissue (including gastric antrum, gastric body, etc.), half of which would be used for pathological section test, and the other half would be used for testing pyloric colonization, immune factors and other related indicators.

### Example 1 Screening of Lactobacillus strains antagonistic to H.Pylori

### (1) Experimental materials and methods of use

*Helicobacter pylori* is *H.Pylori SS* from the NTCC National Type Culture Collection. When using *H.Pylori,* a small amount of the bacterial solution in the bacteria preservation tube was dipped using an inoculation loop, streaked on a Columbia blood agar plate (containing 7.5% of sterile defibrinated sheep blood), and cultured in a three-gas incubator (85% N₂, 10% CO₂, 5% O₂) at 37°C for 3 days. A single colony on the surface of the plate was picked, inoculated into the liquid BHI medium (containing 5% of fetal bovine serum) and cultured for 4 days, and then inoculated into fresh BHI medium with an inoculum volume of 2% and cultured for 4 days before use.

Human gastric adenocarcinoma cells (AGS) were purchased from the Shanghai Cell Bank of the Chinese Academy of Sciences. When using AGS cells, they were resuspended in fetal bovine serum containing 10% DMSO, gradient cooled, and then frozen in liquid nitrogen. When using AGS cells, 10 mL of F-12 medium containing 5% fetal bovine serum was added to the culture dish, centrifuging the cryogenic vials, discarding the supernatant, adding the culture medium to resuspend the cells, pouring the resuspended cells into the culture dish to disperse them evenly and placing them in a 37°C incubator containing 5% CO₂ for culture . When the cells covered the monolayer, they are passaged at a ratio of 1 to 3, and the cells after two passages were set aside for use.

*Lactobacilli* are derived from fermented foods, dairy products, or human saliva and feces, and are purchased from the Guangdong Microbial Culture Collection Center and Biobw strain library, respectively. When using *Lactobacillus,* it was first streaked on the MRS plate and cultured at 37°C for two days. A single colony was picked, inoculated into the MRS liquid tube and cultured for 18 hours, and inoculated into fresh MRS culture medium with an inoculum volume of 2% and cultured for 18 hours before use.

(2) Determination of the antibacterial effect of *Lactobacillus* on the growth of *H.Pylori*: the concentration of the activated two-generation *H.Pylori* bacterial suspension was adjusted to 1*10⁸ CFU/mL, taking 100 µL of the adjusted suspension and spreading it evenly on the Columbia blood agar plate, placing and gently pressing the Oxford cup when the surface of the culture medium is dry, adding 150 µL of *Lactobacillus* suspension (1*10⁸ CFU/mL), setting a blank control (100 µL of MRS liquid culture medium of pH 6.2), and placing them at 4°C for diffusion for 4 hours. After diffusion, they were cultured in a three-gas incubator for 72 hours. The antibacterial results were observed and the diameter of the inhibition zone was measured using a vernier caliper.

(3) Determination of the inhibitory ability of *Lactobacillus* on *H.Pylori* adhesion to AGS cells: AGS cells were inoculated in a 96-well plate (2×104 cells/well) and cultured overnight. After the cells were in an adherent state, they were washed three times with PBS to remove dead cells, then adding *H.Pylori* and *Lactobacillus* resuspended in F-12 medium (without serum) at a multiplicity of infection (MOI) of 100 to co-culture for 2 hours respectively, washing three times with PBS to remove unadherent *Lactobacillus* and *H.Pylori,* finally adding 200 µL of urease reagent (0.9% NaCl, 14 µg/mL phenol red, 20 mmol/L urea, pH 6.8) and culturing for 3 hours, and measuring the absorbance value at the wavelength of 550 nm.

**Table 1 Comparison of antibacterial effects and adhesion rates of different Lactobacilli**

| Strain number | Accession number | Species | Inhibition zone/mm | Adhesion rate/% |
|---|---|---|---|---|
| blank | blank | / | 0 | 102.18±6.36 |
| PC-B1 | GDMCC 1.140 | *Lactobacillus plantarum* | 14.27±0.62 | 93.48±7.12 |
| PC-B2 | GDMCC 1.1516 | *Lactobacillus plantarum* | 11.26±0.89 | 108.25±5.49 |
| PC-B3 | GDMCC 1.2685 | *Lactobacillus plantarum* | 14.76±0.72 | 84.18±4.22 |
| NL-L 1 | GDMCC 1.1732 | *Lactobacillus rhamnosus* | 12.34±0.56 | 113.36±8.55 |
| NL-L2 | GDMCC 1.2868 | *Lactobacillus plantarum* | 10.57±0.56 | 107.12±4.93 |
| NL-L3 | GDMCC 1.1798 | *Lactobacillus rhamnosus* | 11.69±0.64 | 101.34±4.77 |
| NL-L4 | Bio-03636 | *Lactobacillus paracasei* | 11.65±0.62 | 95.54±4.78 |
| SMT-F1 | GDMCC 1.2885 | *Lactobacillus casei* | 8.53±0.79 | 103.56±5.98 |
| SMT-F2 | GDMCC 1.648 | *Lactobacillus plantarum* | 12.63±1.18 | 86.77±6.53 |
| CCFM1259 | GDMCC 62419 | *Lactobacillus rhamnosus* | 17.51±1.03* | 68.27±4.65^{#} |
| FB-A3 | Bio-03656 | *Lactobacillus paracasei* | 14.58±1.16 | 66.53±6.32^{#} |
| FB-A4 | GDMCC 1.1797 | *Lactobacillus plantarum* | 9.99±0.46 | 99.32±8.51 |
| FB-O1 | GDMCC 1.410 | *Lactobacillus casei* | 17.28±0.71* | 84.68±5.65 |
| FB-O2 | GDMCC 1.2223 | *Lactobacillus rhamnosus* | 14.75±0.74 | 72.78±6.53^{#} |
| FB-O3 | GDMCC 1.325 | *Lactobacillus rhamnosus* | 13.37±0.51 | 84.73±5.66 |
| FB-O4 | Bio-67299 | *Lactobacillus plantarum* | 8.51±0.43 | 104.23±7.72 |
| FB-O5 | GDMCC 1.411 | *Lactobacillus casei* | 11.81±0.93 | 88.74±6.65 |
| TY-O1 | GDMCC 1.80 | *Lactobacillus paracasei* | 16.75±0.72* | 73.13±5.22^{#} |
| TY-O3 | GDMCC 1.191 | *Lactobacillus plantarum* | 10.18±0.46 | 90.56±6.23 |
| TY-O4 | Bio-67238 | *Lactobacillus rhamnosus* | 15.36±0.73* | 83.57±6.29 |
| SN-L1 | Bio-03644 | *Lactobacillus casei* | 13.65±1.11 | 88.55±7.91 |
| SN-L2 | Bio-79975 | *Lactobacillus rhamnosus* | 9.82±0.68 | 107.52±6.84 |
| SN-L3 | GDMCC 1.380 | *Lactobacillus plantarum* | 15.84±1.28* | 76.35±6.28^{#} |
| SN-L4 | Bio-67203 | *Lactobacillus rhamnosus* | 13.25±0.77 | 86.52±3.56 |
| SN-L5 | GDMCC 1.1799 | *Lactobacillus paracasei* | 8.27±0.49 | 96.77±5.62 |
| NJ-F1 | Bio-67166 | *Lactobacillus casei* | 8.96±0.52 | 88.72±6.39 |
| NJ-F2 | Bio-84643 | *Lactobacillus rhamnosus* | 13.56±0.68 | 104.67±8.82 |
| NJ-F3 | GDMCC 1.159 | *Lactobacillus paracasei* | 16.84±1.19* | 82.81±6.73 |
| NJ-F6 | Bio-67229 | *Lactobacillus rhamnosus* | 13.34±1.05 | 94.57±4.53 |
| NJ-F7 | Bio-60181 | *Lactobacillus plantarum* | 11.81±0.58 | 96.77±4.96 |

| | | | | |
|---|---|---|---|---|
| Note: * represents the value> 15.00mm; # represents the value< 80.00%. | | | | |

### (4) The antibacterial effect of Lactobacillus on the growth of H.Pylori

30 strains of *Lactobacillus* were used as the starting strains in the experiment. As shown in Table 1, by comparing the size of the inhibition zones against *H.Pylori,* it is found that the inhibition zones of 30 strains are 8.27-17.51 mm, with 6 strains having inhibition zones greater than 15mm against *H.Pylori,* indicating a stronger antibacterial effect than other strains. The 6 strains are ranked according to their inhibition zone size as follows: CCFM1259, FB-O1, NJ-F3, TY-O1, SN-L3, and TY-O4.

### (5) Inhibitory effect of Lactobacillus on H.Pylori adhesion to AGS cells

The inhibitory effect of 30 strains of Lactobacillus on *H.Pylori* adhesion to AGS cells were compared in the experiment. As shown in Table 1, the results show that the ability of different Lactobacilli to reduce the adhesion of *H.Pylori* varies greatly. After treatment with different Lactobacilli, The adhesion rates of *H.Pylori* range from 66.53% to 113.36%, with 5 strains having the adhesion rates of *H.Pylori* less than 80%. According to their ability to inhibit *H.Pylori* adhesion, the strain numbers of the 5 strains are: FB-A3, CCFM1259, FB-O2, TY-O1, and SN-L3.

### (6) Selection of Lactobacillus strains antagonistic to H.Pylori

Taking into account the ability of Lactobacillus to inhibit the growth of *H.Pylori* and the ability to inhibit the adhesion of *H.Pylori* to AGS cells, there are three strains that can inhibit the growth of *H.Pylori* while having a good effect in reducing the adhesion rate. According to the ability to inhibit the adhesion rate, the strains are ranked as follows: *Lactobacillus rhamnosus* CCFM1259, Lactobacillus paracasei TY-O1 and Lactobacillus plantarum SN-L3. Among them, the inhibition zone of CCFM1259 is 0.76 mm and 1.67 mm larger than that of the other two strains, and the adhesion rate is 4.86% and 8.08% lower respectively. Therefore, *Lactobacillus rhamnosus* CCFM1259 is preferred in the experiment as a Lactobacillus strain that antagonizes *H.Pylori.*

### Example 2 Evaluation of the animal efficacy of the combination of HA and Lactobacillus rhamnosus against Helicobacter pylori

After several C57BL/6 mice were fed normally for one week, they were randomly divided into 5 groups (n=10): blank group, model group, and intervention group. For the intervention group, HA (HA with an average molecular weight of 1400 kDa this time), *Lactobacillus rhamnosus* CCFM1259 (1*10⁹ CFU/mL) and their compounding product was administered by gavage for the first time respectively, and *H.Pylori* (1*10⁹ CFU/mL) resuspended in BHI was administered by gavage after an interval of 1 hour. The mice in the blank group and the model group were administered the same amount of PBS by gavage for the first gavage, and administered the same amount of BHI by gavage after an interval of 1 hour. Experimental mice were administered by gavage once every other day. The weight changes of the experimental mice were measured regularly; after the experiment, the pyloric colonization amount in the stomach of the mice, the levels of anti-pyloric antibodies and cytokines (IL-8 and TNF-α) in the serum were measured, and the histopathology section of the gastric antrum and stomach body was performed. The mice were given normal drinking water and standard feed.

The specific treatment scheme for experimental animals is shown in Table 2.

**Table 2 Experimental animal treatment scheme 1 (n=10)**

| Treatment method | Days 1, 3, 5, 7. and 9 | |
|---|---|---|
| | First time | Second time |
| Blank group | Administering PBS by gavage, 0.2mL/animal/day | Administering BHI by gavage, 0.2mL/animal/day |
| Model group (*H.Pylori*) | Administering PBS by gavage, 0.2mL/animal/day | Administering 0.2Ml of *H.Pylori* resuspended in BHI (1*10⁹CFU/mL) |
| HA group (HA+*H.Pylori*) | Administering HA by gavage (3 g/L), 0.2mL/animal/day | |
| CCFM1259 group *(Lactobacillus rhamnosus* CCFM1259+*H.Pylori*) | Administering *Lactobacillus rhamnosus* CCFM1259 by gavage (1*10⁹CFU/mL), 0.2mL/animal/day | |
| Compounding group *(HA+Lactobacillus rhamnosus CCFM1259+H.Pylori)* | Mixing HA (3 g/L) and *Lactobacillus rhamnosus* CCFM1259 (1 * 10⁹ CFU/mL) evenly at a volume ratio of 1:1 and administering by gavage, 0.2mL/animal/day | |

| | | |
|---|---|---|
| Note: BHI is brain heart infusion broth medium. There was an hour interval between the first and second administration by gavage every day. | | |

HA group: the amount of sodium hyaluronate was based on 200 mg/day/person (person was calculated as 60 kg), converted into the recommended dose for mice: 9.1*200mg/60 kg=30mg/kg (mice was calculated as 20g), the concentration of sodium hyaluronate solution was 3g/L based on the volume of 0.2mL by gavage.

HA+CCFM1259 group: the dosage of sodium hyaluronate and *Lactobacillus rhamnosus* was reduced by half compared with the HA group and CCFM1259 group.

### (1) Changes in body weight of mice during the experiment

As can be seen from Table 3, the body weight of the mice is maintained at about 20g during the experiment. There is no significant difference in the body weight of the mice between each group. There is also no significant change in the body weight of the mice before and after the intervention. That is, *H.Pylori* infection and compounding formula intervention do not have a significant impact on the body weight of the mice.

**Table 3 Changes in body weight of mice in each group during the experiment (Mean±SD)**

| Group | body weight on week 0/g | body weight on week 1/g | body weight on week 2/g |
|---|---|---|---|
| Blank group | 18.22±1.52 | 19.67±1.57 | 21.06±1.73 |
| Model group | 18.50±0.74 | 19.80±1.25 | 21.52±1.38 |
| HA group | 18.67±0.46 | 20.18±1.01 | 21.29±1.97 |
| CCFM1259 group | 18.48±0.66 | 19.87±1.12 | 21.43±1.25 |
| Compounding group | 18.59±0.30 | 20.09±0.93 | 21.40±1.44 |

### (2) Effects of different treatments on H.Pylori colonization amount

The change in the *H.Pylori* colonization amount in the stomach of mice is the most direct indicator that reflects the effect of the intervention formula on *H.Pylori* infection. It can be seen from Figure 1 that the content of *H.Pylori* in the stomach of mice in the model group is approximately 236 times that of the blank group, and the *H.Pylori* colonization amount in the model group increases significantly (p<0.01), indicating that the modeling is successful. After treatment with HA (p<0.01), *Lactobacillus rhamnosus* CCFM1259 (p<0.05) and the compounding formula (p<0.001), the *H.Pylori* colonization amount in the stomach of infected mice is significantly reduced; compared with the model group, the average Log value of *H.Pylori* colonization amount decreases by approximately 34.75%, 29.33% and 52.64% respectively. In addition, the compounding group is significantly better than the HA group and CCFM1259 group (p<0.05). Compared with the HA group and CCFM1259 group, the average log value of the *H.Pylori* colonization amount in the compounding group decreases by 27.41% and 32.97% respectively. In summary, it shows that the intervention effect of the compounding formula is more obvious than that of using HA and *Lactobacillus rhamnosus* CCFM1259 alone, and *Lactobacillus rhamnosus* CCFM1259 and HA have a synergistic effect in reducing the *H.Pylori* colonization amount.

### (3) Improvement of gastric pathology with different treatments

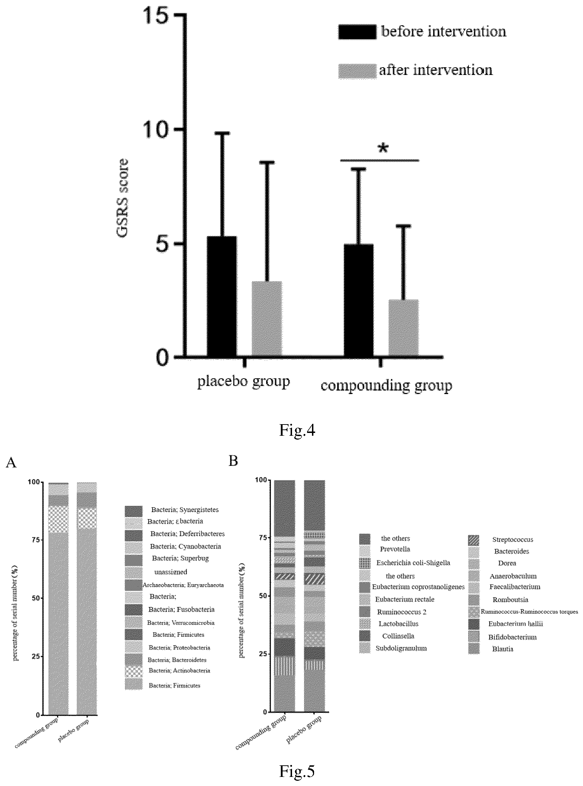

The results of HE staining of the gastric mucosa of mice in different treatment groups are shown in Figure 2. After infection with *H.Pylori,* the infiltration of inflammatory cells (lymphocytes and eosinophils) in the lamina propria of the gastric mucosa of mice increases significantly (Figure 2B). After treatment with the HA group (Figure 2C), the *Lactobacillus rhamnosus* CCFM1259 group (Figure 2D) and the compounding formula (Figure 4E), the inflammatory response caused by *H.Pylori* infection is significantly improved. Compared with the HA group (Figure 2C) and the *Lactobacillus rhamnosus* CCFM1259 group (Figure 2D), the improvement effect on the gastric mucosa of mice after treatment with the compounding formula (Figure 2E) is more significant, and there is basically no inflammatory cell infiltration. It is further proved that *Lactobacillus rhamnosus* CCFM1259 and HA have a synergistic effect in antagonizing the inflammatory response caused by *H.Pylori* infection.

It can be seen from the results of this example that HA, *Lactobacillus rhamnosus* CCFM1259 and the compounding formula can significantly reduce the *H.Pylori* colonization amount in the stomach of mice, and significantly improve the inflammatory response caused by *H.Pylori* infection. The compounding formula has a more significant effect on reducing the *H.Pylori* colonization amount and improving the gastric mucosa of mice compared with HA alone and probiotics alone. There is basically no inflammatory cell infiltration in the lamina propria of the gastric mucosa of mice, indicating that *Lactobacillus rhamnosus* CCFM1259 and HA has a synergistic effect in antagonizing the inflammatory response caused by *H.Pylori* infection and reducing the *H.Pylori* colonization amount in the stomach of mice.

### Example 3 Evaluation of the effect of compounding HA with different molecular weights and Lactobacillus rhamnosus on Helicobacter pylori infection in vivo

After several C57BL/6 mice were fed normally for one week, they were randomly divided into 7 groups (n=10): blank group, model group, and intervention group. For the intervention group, a composition obtained by mixing HA with average molecular weights of 1200 kDa, 1400 kDa, 1600 kDa, 1800 and 2000 kDa, respectively, and *Lactobacillus rhamnosus* CCFM1259 (1*10⁹ CFU/mL) at a volume ratio of 1:1, was administered by gavage for the first time, and *H.Pylori* (1*10⁹ CFU/mL) resuspended in BHI was administered by gavage after an interval of 1 hour. The mice in the blank group and the model group were administered the same amount of PBS by gavage for the first gavage, and administered the same amount of BHI by gavage after an interval of 1 hour. Experimental mice were administered by gavage once every other day. The weight changes of the experimental mice were measured regularly; after the experiment, the pyloric colonization amount in the stomach of the mice was measured. The mice were given normal drinking water and standard feed.

The specific treatment scheme for experimental animals is shown in Table 4.

**Table 4 Experimental animal treatment scheme 2 (n=10)**

| Treatment method | Days 1, 3, 5, 7, and 9 | |
|---|---|---|
| | First time | Second time |
| Blank group | Administering PBS by gavage, 0.2mL/animal/day | Administering BHI by gavage, 0.2mL/animal/day |
| Model *group*(*H.Pylori*) | Administering PBS by gavage, 0.2mL/animal/day | Administering 0.2mL of *H.Pylori* resuspended in BHI (1*10⁹CFU/mL) |
| Intervention group: composition 1 (1200 kDa *HA+Lactobacillus rhamnosus* CCFM1259+*H.Pylori*) | Administering composition 1 by gavage, 0.2mL/animal/day | |
| Intervention group: composition 2 (1400 kDa *HA+Lactobacillus rhamnosus* CCFM1259+*H.Pylori*) | Administering composition 1 by 2, 0.2 mL/animal/day | |
| Intervention group: composition 3 (1600 kDa *HA+Lactobacillus rhamnosus* CCFM1259+*H.Pylori*) | Administering composition 3 by gavage, 0.2 mL/animal/day | |
| Intervention group: composition 4 (1800 kDa *HA+Lactobacillus rhamnosus* CCFM1259+*H.Pylori*) | Administering composition 4 by gavage, 0.2mL/animal/day | |
| Intervention group: | Administering composition 5 | |
| composition 5 (2000 kDa *HA+Lactobacillus rhamnosus* CCFM1259+*H.Pylori*) | by gavage, 0.2mL/animal/day | |

| | | |
|---|---|---|
| Note: BHI is brain heart infusion broth medium. There was an hour interval between the first and second administration by gavage every day. | | |

The amount of sodium hyaluronate was based on 200 mg/day/person (person was calculated as 60 kg), converted into the recommended dose for mice: 9.1*200mg/60 kg=30mg/kg (mice was calculated as 20g), the concentration of sodium hyaluronate solution was 3g/L based on the volume of 0.2mL by gavage.

After HA and *Lactobacillus rhamnosus* were compounded at 1:1, the concentration of HA was 1.5g/L and the concentration of *Lactobacillus rhamnosus* was 5*10⁸ CFU/mL.

### (1) Changes in survival rate and body weight of mice during the experiment

As can be seen from Table 5, the body weight of the mice is maintained at about 20g during the experiment. There is no significant difference in the body weight of the mice between each group. There is also no significant change in the body weight of the mice before and after the intervention. That is, *H.Pylori* infection and compounding formula intervention do not have a significant impact on the body weight of the mice.

**Table 5 Changes in body weight of mice in each group during the experiment (Mean±SD)**

| Group | body weight on week 0/g | body weight on week 1/g | body weight on week 2/g |
|---|---|---|---|
| Blank group | 18.77±1.36 | 19.72±1.53 | 21.24±1.66 |
| Model group | 18.58±1.45 | 19.68±1.63 | 21.53±1.72 |
| Composition 1 (1200 kDa HA+CCFM1259 group) | 18.69±1.26 | 19.83±1.42 | 21.48±1.56 |
| Composition 2 (1400 kDa HA+CCFM1259 group) | 18.52±1.42 | 19.98±1.27 | 21.32±1.25 |
| Composition 3 (1600 kDa HA+CCFM1259 group) | 18.73±1.51 | 20.02±1.56 | 21.65±1.48 |
| Composition 4 (1800 kDa HA+CCFM1259 group) | 18.70±1.38 | 20.15±1.55 | 21.36±1.61 |
| Composition 5 (2000 kDa HA+CCFM1259 group) | 18.55±1.46 | 20.06±1.39 | 21.45±1.36 |

### (2) Effects of compounding of HA with different molecular weights and Lactobacillus rhamnosus on H.Pylori colonization amount

As can be seen from Figure 3: Compared with the blank group, the *H.Pylori* colonization amount in the model group increases significantly (p<0.01), and the average value is approximately 155 times that of the blank group, indicating that the modeling is successful. After treatment with HA with average molecular weights of 1200 kDa, 1400 kDa, 1600 kDa, 1800 kDa and 2000 kDa compounded with *Lactobacillus rhamnosus* CCFM1259, the *H.Pylori* colonization amount in the stomach of mice can be significantly reduced (p<0.05); compared with the model group, the mean log values of *H.Pylori* colonization amount decrease by 31.93%, 47.83%, 53.92%, 49.16% and 34.76% respectively. Especially after compounding HA with average molecular weights of 1400 kDa, 1600 kDa and 1800 kDa, and *Lactobacillus rhamnosus* CCFM1259, the *H.Pylori* colonization amount in the stomach is very significantly reduced (p<0.001), and the effect is close to that of the blank group.

It can be seen from the results of this example that, compared with the model group, after H Pylori-infected mice are administered a compounding product of HA with an average molecular weight of 1400-1800 kDa and *Lactobacillus rhamnosus* CCFM1259 by gavage, *H.Pylori* colonization amount in the stomach is significantly reduced (p<0.01), and the average Log value of *H.Pylori* colonization amount can be reduced by 47.83%-53.92%. The effect is more significant than that of the compounding products of HA with the molecular weights of 1200 kDa and 2000 kDa, and is close to the normal level.

### Example 4 Clinical trial efficacy evaluation of hyaluronic acid-Lactobacillus rhamnosus composition against Helicobacter pylori

### 4.1 Population for study

*40 Helicobacter pylori* infection-positive patients (28-65 years old, half male and half female) were recruited in the clinical study. Diagnostic criteria were passing ¹⁴C breath test, rapid urease test, or histological examination. Volunteers were required to have not received anti-pyloric treatment in the past, and have not taken antibiotics or probiotic products one month before inclusion in the study; have not undergone gastrointestinal surgery; must take products in strict accordance with the requirements during the experiment, and cannot take antibiotics or other probiotic products.

### 4.2 Clinical study design

### (1) Experimental products

The probiotic compounding products, hyaluronic acid products and placebo products in the experimental products were all food grade products and can be taken directly or with warm water (not exceeding 37°C), and provided by the Food Biotechnology Center of the School of Food Science and Technology, Jiangnan University. The products were all powders with the same appearance and packaging.
A: HA-probiotic compounding product: ① *Lactobacillus rhamnosus* freeze-dried bacterial powder (1×10¹¹ CFU/g bacterial powder) 50mg/strip; ② Sodium hyaluronate with an average molecular weight of 1600 kDa 100mg/strip; ③ Inulin 300mg/strip; ④ Galacto-oligosaccharide 150 mg/strip; ⑤ Isomaltooligosaccharide 80 mg/strip; ⑥ Maltodextrin 1320 mg/strip.
B: placebo product: ① Inulin 300mg/strip; ② Galacto-oligosaccharide 150mg/strip; ③ Isomaltooligosaccharide 80mg/strip; ④ Maltodextrin 1470mg/strip.

### (2) Experimental design and subject grouping

The experimental design was a double-blind, parallel randomized controlled trial.

After 40 subjects were recruited, they were divided into groups. 40 patients with *Helicobacter pylori* infection were randomly divided into two groups using computer software to generate a random number sequence, namely the placebo group and the hyaluronic acid-probiotic compounding group, with 20 people in each group. Each group took bacterial powder twice a day (except for the different ingredients, the appearance and packaging of the products in the placebo group and the compounding group were the same, with no obvious difference).

### (3) Study period

Since it takes a certain amount of time for probiotics to develop their physiological properties, the study period was 10 weeks. It comprised a 2-week experimental preparation period, a 4-week probiotic administration period, and a 4-week follow-up observation period.

### (4) Indicator measurement

¹⁴C Breath test, scale indicators (GSRS table), intestinal flora distribution.

### 4.3 Clinical trial results

### (1) Basic information of clinical subjects

**Table 6 Basic information of enrolled subjects**

| Group | Number of people | Gender (Male/Female) | Age |
|---|---|---|---|
| Placebo | 16 | 5/11 | 48.69±16.87 |
| Compounding group | 17 | 6/11 | 43.24±12.73 |

A total of 40 *H.Pylori*-positive patients who met the requirements were recruited for the clinical trial. Among them, 7 dropped out and were lost to follow-up. Table 6 shows the basic information such as gender and age of the two groups of participants. There is no significant difference in the baseline conditions of the subjects in the two groups.

### (2) The impact of different intervention methods on subjects' ¹⁴C expiratory value

The determined ¹⁴C expiratory value can characterize the patient's *H.Pylori* infection. The experimental results in Tables 7 and 8 show that after taking the compounding formula, the negative conversion rate and effective rate of *Helicobacter pylori* patients reach 70.59% and 82.35% respectively. Compared with the placebo group, the negative conversion rate and effective rate increases 125.88% and 119.60% respectively.

**Table 7 Effect of compounding formula on the negative conversion rate of H.Pylori subjects**

| Group | N | Negative | Positive | Negative conversion rate % |
|---|---|---|---|---|
| Placebo | 16 | 5 | 11 | 31.25 |
| Compoundin g group | 17 | 12 | 5 | 70.59 |

**Table 8 Effect of compounding formula on subjects' anti- H.Pylori effective rate**

| Group | N | Effective | Invalid | Effective rate% |
|---|---|---|---|---|
| Placebo | 16 | 6 | 10 | 37.50 |
| Compoun ding group | 17 | 14 | 3 | 82.35 |

### (3) The impact of different intervention methods on subjects' gastrointestinal symptoms

GSRS is an important indicator of the severity and frequency of gastrointestinal symptoms in clinical patients. The lower the score, the milder the symptoms. The subjects filled it out one day before the start of the experiment and one day after the end. According to the statistical results in Figure 4, there is no significant change in adverse gastrointestinal symptoms in the placebo group before and after intervention (p>0.05); however, after the intervention with the HA-probiotic compounding group, the gastrointestinal symptoms of the subjects are improved significantly (p<0.05).

### (4) The impact of different intervention methods on the composition and diversity of the intestinal flora of subjects

Compared with the placebo group, the composition of the intestinal flora of the subjects changes after intervention with the HA-probiotic compounding formula.

### Phylum level analysis:

As shown in Figure 5A, the intestinal flora is mainly composed of *Firmicutes*, *Actinobacteria*, *Bacteroidetes*, and *Proteobacteria.* Compared with the placebo group, the proportion of *Actinobacteria* in the fecal intestinal flora of the compounding group increases.

### Genus level analysis:

Furthermore, the fecal intestinal flora of the two groups were analyzed at the genus level. The results are shown in Figure 5B. Compared with the placebo group, the compounding group reduces the abundance of *Streptococcus*, *Ruminococcus*, and the conditional pathogenic bacteria *Escherichia coli-Shigella* and other flora, and increases the abundance of beneficial bacteria, such as *Lactobacillus* and *Bifidobacterium.*

It can be seen from the results of the above examples that after the intervention of the prebiotic composition of HA and *Lactobacillus rhamnosus,* the gastrointestinal symptoms of the subjects are significantly improved (GSRS score, p<0.05), and the patient's negative conversion rate and effective rate are also increased by multiples, indicating that the composition of the present application has a significant effect in antagonizing *H.Pylori* infection in the stomach. After the intervention with the compounding product, the negative conversion rate and effective rate of *H.Pylori* patients reach 70.59% and 82.35% respectively, which are 2.126 times and 2.120 times higher than those of the placebo group. In addition, the compounding group also has the function of improving the composition and diversity of intestinal flora, reducing the proportion of intestinal pathogenic bacteria, increasing the abundance of beneficial bacteria *Lactobacillus* and *Bifidobacterium,* which is beneficial to maintain the patient's gastrointestinal health.

Although the embodiments of the present application have been described above, the present application is not limited to the above-mentioned specific embodiments and application fields. The above-mentioned specific embodiments are only illustrative and instructive, rather than restrictive. Under the inspiration of this description and without departing from the scope of protection of the claims of the application, those of ordinary skill in the art can also make many forms, which all belong to the scope of protection of the application.

## Claims

1. A strain of *Lactobacillus rhamnosus,* wherein the *Lactobacillus rhamnosus* is deposited in the Guangdong Microbial Culture Collection Center with an accession number of GDMCC NO.62419.

2. Use of the *Lactobacillus rhamnosus* according to claim 1 in inhibiting *Helicobacter pylori.*

3. A composition for inhibiting *Helicobacter pylori,* comprising a hyaluronic acid or a salt thereof and *Lactobacillus rhamnosus.*

4. The composition according to claim 3, wherein the salt of the hyaluronic acid is any one or two or more of sodium salt, potassium salt, magnesium salt, calcium salt, zinc salt and bismuth salt of the hyaluronic acid,
preferably, the average molecular weight of the hyaluronic acid or a salt thereof is 1200-2000 kDa, more preferably 1400-1800 kDa.

5. The composition according to any one of claims 3-4, wherein the composition can provide the hyaluronic acid or a salt thereof with an amount of more than or equal to 80 mg/day/person (a person is calculated as 60 kg), or
the viable count of *Lactobacillus rhamnosus* is more than or equal to 1×10⁸ cfu/day/person (a person is calculated as 60 kg);
preferably, the composition can provide the hyaluronic acid or a salt thereof with an amount of more than or equal to 100 mg/day/person (a person is calculated as 60 kg), or
the viable count of *Lactobacillus rhamnosus* is more than or equal to 1×10⁹ cfu/day/person (a person is calculated as 60 kg).

6. The composition according to any one of claims 3-5, wherein the composition further comprises an adjuvant,
preferably, the adjuvant comprises one or two or more of inulin, galactooligosaccharides, isomaltooligosaccharides, maltodextrin, erythritol, xylose, arabinose, rhamnose, galactose, fucose, mannose, fructose, sorbose, glucose, glycerol, galactitol, sorbitol, xylitol, mannitol, lactose, maltitol, lactitol, sucrose, trehalose, raffinose, stachyose, oligofructose, oligoxylose, oligomannose, β-glucan, hydroxyethyl starch, resistant dextrin, whey protein, collagen, skimmed milk, pectin, and gelatin.

7. The composition according to any one of claims 3-6, wherein the *Lactobacillus rhamnosus* is the *Lactobacillus rhamnosus* according to claim 1 or 2.

8. Use of the *Lactobacillus rhamnosus* according to claim 1 or 2, or the composition according to any one of claims 3-7 in the preparation of foods or health products inhibiting *Helicobacter pylori.*

9. Use of *Lactobacillus rhamnosus* and hyaluronic acid or a salt thereof in combination in the preparation of foods or health products inhibiting *Helicobacter pylori.*

10. The use according to claim 9, wherein the dosage of the hyaluronic acid or a salt thereof is that a composition comprising the hyaluronic acid or a salt thereof can provide the hyaluronic acid or a salt thereof with an amount of more than or equal to 80 mg/day/person (a person is calculated as 60 kg), or
the dosage of *Lactobacillus rhamnosus* is that the viable count of *Lactobacillus rhamnosus* is more than or equal to 1×10⁸ cfu/day/person (a person is calculated as 60 kg);
preferably, the dosage of the hyaluronic acid or a salt thereof is that a composition comprising the hyaluronic acid or a salt thereof can provide the hyaluronic acid or a salt thereof with an amount of more than or equal to 100 mg/day/person (a person is calculated as 60 kg), or
the dosage of *Lactobacillus rhamnosus* is that the viable count of *Lactobacillus rhamnosus* is more than or equal to 1×10⁹ cfu/day/person (a person is calculated as 60 kg);
preferably, the salt of the hyaluronic acid is any one or two or more of sodium salt, potassium salt, magnesium salt, calcium salt, zinc salt and bismuth salt of hyaluronic acid, and the average molecular weight of the hyaluronic acid or a salt thereof is 1200-2000 kDa, more preferably 1400-1800 kDa;
more preferably, the *Lactobacillus rhamnosus* is the *Lactobacillus rhamnosus* according to claim 1 or 2.
